(19) Europäisches Patentamt / European Patent Office / Office européen des brevets

(11) **EP 2 164 449 B1**

(12) **EUROPÄISCHE PATENTSCHRIFT**

(45) Veröffentlichungstag und Bekanntmachung des Hinweises auf die Patenterteilung:
**25.01.2017** **Patentblatt 2017/04**

(21) Anmeldenummer: **08773438.0**

(22) Anmeldetag: **14.06.2008**

(51) Int Cl.:
*A61K 8/06* (2006.01)     *A61K 8/37* (2006.01)
*A61K 8/60* (2006.01)     *A61K 8/73* (2006.01)
*A61K 8/81* (2006.01)     *A61Q 5/02* (2006.01)
*A61Q 5/12* (2006.01)     *A61Q 19/10* (2006.01)

(86) Internationale Anmeldenummer:
**PCT/EP2008/004801**

(87) Internationale Veröffentlichungsnummer:
**WO 2008/155073 (24.12.2008 Gazette 2008/52)**

(54) **SHAMPOO-ZUSAMMENSETZUNG MIT VERBESSERTER PFLEGELEISTUNG**

SHAMPOO COMPOSITION HAVING IMPROVED CARE PROPERTIES

COMPOSITION DE SHAMPOOING À PERFORMANCES AMÉLIORÉES EN TERMES DE SOINS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität:
19.06.2007  EP 07011967
27.09.2007  DE 102007046575
03.04.2008  DE 102008017032
03.04.2008  DE 102008017034
07.05.2008  DE 102008022433
21.05.2008  DE 102008024570

(43) Veröffentlichungstag der Anmeldung:
**24.03.2010** **Patentblatt 2010/12**

(73) Patentinhaber: **Cognis IP Management GmbH**
**40589 Dusseldorf (DE)**

(72) Erfinder:
• **HLOUCHA, Matthias**
**50677 Köln (DE)**
• **HAAKE, Hans-Martin**
**40699 Erkrath (DE)**
• **MÜLLER, Michael**
**89165 Dietenheim (DE)**
• **KÜSTERS, Esther**
**40625 Düsseldorf (DE)**
• **MENZER, Jasmin**
**40789 Monheim (DE)**
• **EISFELD, Wolf**
**40764 Langenfeld (DE)**
• **SEIPEL, Werner**
**40723 Hilden (DE)**
• **HENSEN, Hermann**
**42781 Haan (DE)**
• **BÖTTCHER, Axel**
**41564 Kaarst (DE)**
• **GEHM, Esther, Ricarda**
**67655 Kaiserslautern (DE)**

(74) Vertreter: **BASF IP Association**
**BASF SE**
**ZRX-C6**
**67056 Ludwigshafen (DE)**

(56) Entgegenhaltungen:
WO-A1-99/53889          DE-A1- 10 241 296
DE-A1-102004 030 885

**Beschreibung**

Die Erfindung befindet sich auf dem Gebiet der kosmetischen Mittel zum Konditionieren von Haut und Haaren, die Mikroemulsionen und Polymere enthalten.

Nach der Wäsche fühlen sich Haut und Haare häufig rauh und spröde an, insbesondere wenn sie schon durch Umwelteinflüsse vorgeschädigt sind. Haare können überdies noch durch Färben oder Dauerwellen geschädigt sein und zeichnen sich dann nach der Haarwäsche oft durch einen trockenen strohigen Griff aus.

Daher werden in Shampoo-Zusammensetzungen häufig Konditionierer eingesetzt, die diesen Nachteilen entgegen wirken sollen. Häufig findet man daher Shampoo-Zusammensetzungen, die als Konditionierer Silikone enthalten. Diese können jedoch irreversibel auf die Haare aufziehen und verursachen so ihrerseits negative Auswirkungen auf den Griff, im schlimmsten Fall sogar zu Problemen beim Färben und Dauerwellen von Haaren.

Weiterhin kommen als Konditionierungsmittel in diesen kosmetischen Zubereitungen Öle und Wachse in Frage. Diese sind in ihrer Wirkung jedoch bei weitem nicht so ausgeprägt wie die o.g. Silikone. Ausserdem sind durch Verwendung dieser Konditionierer nur noch trübe Formulierungen möglich bzw. können diese Öle und Wachse sowieso nur in geringen Mengen in den Zubereitungen stabilisiert werden.

Konditionierende Haarshampoos enthaltend Silikone in Form einer Mikroemulsion sind aus DE 10 2004 030 885 bekannt.

Die Aufgabe der vorliegenden Erfindung hat somit darin bestanden, kosmetische Mittel bereit zu stellen, deren Konditionierungsleistung der von silikonhaltigen Zubereitungen entspricht oder bestenfalls diese sogar noch übertrifft.

Überraschenderweise wurde gefunden, dass eine kosmetische Zubereitung enthaltend (a) mindestens ein Tensid ausgewählt aus anionischen, zwitterionischen oder amphoteren Tensiden, (b) eine bestimmte Mikroemulsion und (c) mindestens ein kationisches Polymer die oben genannte Aufgabe löst.

Durch Einarbeitung einer derartigen Mikroemulsion als Komponente (b) der erfindungsgemäßen Zubereitungen gelingt die transparente und stabile Einarbeitung größerer Mengen von Ölkörpern, die dann synergistisch mit den kationischen Polymeren der Komponente (c) in der Zusammensetzung stabilisiert durch die Tenside der Komponente (a) die hervorragenden konditionierenden Eigenschaften der Zubereitung bewirken.

Tenside

Als Komponente a) können anionische, zwitterionische oder amphotere Tenside enthalten sein. Typische Beispiele für **anionische Tenside** sind Seifen, Alkylbenzolsulfonate, Al-kansulfonate, Olefinsulfonate, Alkylethersulfonate, Glycerinethersulfonate, $\alpha$-Methylestersulfonate, Sulfofettsäuren, Alkylsulfate, Fettalkoholethersulfate, Glycerinethersulfate, Alkylethersulfate, Hydroxymischethersulfate, Monoglycerid(ether)sulfate, Fettsäureamid(ether)-sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Alkyloligoglucosidcarboxylate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Besonders bevorzugt ist eine Kombination von Alkylethersulfat und Cocamidopropylbetain, ganz besonders bevorzugt eine Kombination von Laurethsulfat und Cocamidopropylbetain.

Mikroemulsion

Die Mikroemulsionen der Komponente b) besitzen vorzugsweise eine mittlere Teilchengröße von weniger als 1 $\mu$m. Hergestellt werden diese Emulsionen indem man zunächst in einem ersten Schritt eine Mikroemulsion herstellt, enthaltend mindestens 10 - 20 Gew.-% eines Alkyl(oligo)glycosids der allgemeinen Formel $R^1O$-$[G]_p$ in der $R^1$ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht und 4 - 10 Gew.-% eines Esters von Glycerin mit einer gesättigten oder ungesättigten Fettsäure der Kettenlänge C 12-C22 und 5 - 30 Gew.-% eines Ölkörpers und den Rest auf 100 Gew.-% Wasser.

Unter Mikroemulsionen werden zunächst alle makroskopisch homogenen, optisch transparenten, niedrigviskosen und insbesondere thermodynamisch stabilen Mischungen aus zwei miteinander nicht mischbaren Flüssigkeiten und mindestens einem nichtionischen oder einem ionischen Tensid verstanden. Die mittleren Teilchengrößen der Mikroemulsionen liegen üblicherweise unter 100 nm, sie weisen eine hohe Transparenz auf und sind beim Zentrifugieren bei 2000 UPM für mindestens 30 Minuten gegenüber einer sichtbaren Phasenseparation stabil.

Die Herstellung der Mikroemulsionen erfolgt vorzugsweise einfach durch Vermischen der Ölphase mit den weiteren öllöslichen Inhaltsstoffen, Erwärmen der Ölphase über den Schmelzpunkt aller Bestandteile und anschließender Zugabe der wässrigen tensidhaltigen Phase. Die thermodynamisch stabile Mikroemulsion bildet sich dann spontan, ggf. muss noch etwas gerührt werden.

Die Mikroemulsion enthält als zwingende Bestandteile ein Zuckertensid, und zwar ein Alkyl(oligo)glycosid (im Folgenden auch als "APG" bezeichnet). Alkyl- und/oder Alkenyloligoglycoside im Sinne der vorliegenden Lehre folgen dabei der Formel $R^1O\text{-}[G]_p$ in der $R^1$ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,5 liegt. APGs sind in den Mikroemulsionen gemäß der vorliegenden Erfindung in Mengen zwischen 10 und 20 Gew.-%, jeweils bezogen auf die Gesamtmenge der Mikroemulsion enthalten. Besonders bevorzugt sind dabei Mengen im Bereich von 14 bis 19 Gew.-%.

Weiterhin sind Monoester aus Fettsäuren der Kettenlänge C12-C22 mit Glycerin in den Emulsionen enthalten. Dabei werden insbesondere Monoester von Glycerin mit ungesättigten linearen Fettsäuren eingesetzt.

Besonders bevorzugt im Sinne der Erfindung ist Glycerinmonooleat. Diese Glycerinester sind in den Mikroemulsionen in Mengen von 4 bis 10 Gew.-%, vorzugsweise 5 bis 9 Gew.-% - jeweils bezogen auf das Gesamtgewicht der Mikroemulsion - enthalten.

Schließlich enthalten die Mikroemulsionen noch einen Ölkörper, also eine nichtwasserlösliche organische Phase in Mengen von 5 bis 30 Gew.-%. Dabei sind besonders bevorzugte Ölphasen ausgewählt aus der Gruppe von Guerbetalkoholen auf Basis von Fettalkoholen mit 6 bis 18 C-Atomen, Estern linearer $C_6$-$C_{22}$-Fettsäuren mit linearen oder verzweigten $C_6$-$C_{22}$-Fettalkoholen bzw. Estern von verzweigten $C_6$-$C_{13}$-Carbonsäuren mit linearen oder verzweigten $C_6$-$C_{22}$-Fettalkoholen, Estern von linearen $C_6$-$C_{22}$-Fettsäuren mit verzweigten Alkoholen, Estern von $C_6$-$C_{22}$-Fettalkoholen und/oder Guerbetalkoholen mit aromatischen Carbonsäuren, Triglyceriden auf Basis $C_6$-$C_{10}$-Fettsäuren, Estern von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Ölen, verzweigten primäre Alkoholen, substituierten Cyclohexanen, linearen und verzweigten $C_6$-$C_{22}$-Fettalkoholcarbonaten, Guerbetcarbonaten auf Basis von Fettalkoholen mit 6 bis 18, vorzugsweise 8 bis 10 C Atomen, Estern der Benzoesäure mit linearen und/oder verzweigten $C_6$-$C_{22}$-Alkoholen, linearen oder verzweigten, symmetrischen oder unsymmetrischen Dialkylethern mit 6 bis 22 Kohlenstoffatomen pro Alkylgruppe, Ring-öffnungsprodukten von epoxidierten Fettsäureestern mit Polyolen, aliphatischen bzw. naphthenischen Kohlenwasserstoffen und/oder Dialkylcyclohexanen.

Als Ölkomponente können jedoch auch feste Fette und/oder Wachse verwendet werden. Diese können auch in Mischung mit den im vorherigen Abschnitt genannten Ölen vorliegen. Typische Beispiele für Fette sind Glyceride, d.h. feste oder flüssige pflanzliche oder tierische Produkte, die im Wesentlichen aus gemischten Glycerinestern höherer Fettsäuren bestehen. Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Tocopherole und ätherische Öle eignen sich ebenfalls als Ölkomponente.

Als Kohlenwasserstoffe werden organische Verbindungen bezeichnet, die nur aus Kohlenstoff und Wasserstoff bestehen. Sie umfassen sowohl cyclische als auch acyclische (=aliphatische) Verbindungen. Sie umfassen sowohl gesättigte wie einfach oder mehrfach ungesättigte Verbindungen. Die Kohlenwasserstoffe können linear oder verzweigt sein. Je nach Anzahl der Kohlenstoffatome im Kohlenwasserstoff kann man die Kohlenwasserstoffe einteilen in ungradzahlige Kohlenwasserstoffe (wie beispielsweise Nonan, Undecan, Tridecan) oder geradzahlige Kohlenwasserstoffe (wie beispielsweise Octan, Dodecan, Tetradecan). Je nach Art der Verweigung kann man die Kohlenwasserstoffe einteilen in lineare (= unverzweigte) oder verzweigte Kohlenwasserstoffe. Gesättigte, aliphatische Kohlenwasserstoffe werden auch als Paraffine bezeichnet.

Als "Kohlenwasserstoff Gemisch" werden Mischungen von Kohlenwasserstoffen verstanden, die bis zu 10 Gew.-% Substanzen enthalten, die nicht zu den Kohlenwasserstoffen zählen. Die Gew.-% Angaben der linearen C11 und linearen C13 Kohlenwasserstoffe beziehen sich jeweils auf die Summe der im Gemisch vorhandenen Kohlenwasserstoffe. Die bis zu 10 Gew.-% vorhandenen Nicht-Kohlenwasserstoffe werden für diese Berechnung nicht berücksichtigt.

Bei den Substanzen, die nicht zu den Kohlenwasserstoffen zählen und die bis zu 10 Gew.%, insbesondere bis zu 8 Gew.-%, vorzugsweise bis zu 5 Gew.-% im KohlenwasserstoffGemisch enthalten sein können, handelt sich beispielsweise um Fettalkohole, die als nicht umgesetzte Edukte im Kohlenwasserstoff Gemisch verbleiben.

Der Begriff "CX-Kohlenwasserstoff" umfasst Kohlenwasserstoffe mit einer C-Zahl von X, so umfasst beispielsweise der Begriff C11-Kohlenwasserstoff alle Kohlenwasserstoffe mit einer C-Zahl von 11.

Bevorzugt werden Kohlenwasserstoff-Gemische, wobei das Gemisch enthält

(a) 50 bis 90 Gew.-% lineare C-11 Kohlenwasserstoffe, vorzugsweise n-Undecan
(b) 10 bis 50 Gew.-% lineare C13 Kohlenwasserstoffe, vorzugsweise n-Tridecan

bezogen auf die Summe der Kohlenwasserstoffe.

Weiterhin ist ein Kohlenwasserstoff-Gemisch bevorzugt, das mindestens 2 voneinander verschiedene Kohlenwasserstoffe enthält, deren Kohlenstoff Zahl sich um mehr als 1 unterscheidet, wobei diese 2 voneinander verschiedene Kohlenwasserstoffe mindestens 60 Gew.-%, vorzugsweise mindestens 70 Gew.-% - bezogen auf die Summe der Kohlenwasserstoffe ausmachen.

Der Begriff "2 voneinander verschiedene Kohlenwasserstoffe" bezeichnet Kohlenwasserstoffe mit einer unterschiedlichen C-Zahl.

Das bedeutet, wenn das Kohlenwasserstoff Gemisch einen Kohlenwasserstoff mit einer C Zahl von n (n = ganzzahlige Zahl) enthält, so enthält das Gemisch mindestens noch einen weiteren Kohlenwasserstoff mit einer C-Zahl von größer gleich n+2 bzw. kleiner gleich n-2.

Vorzugsweise ist n eine ungrade Zahl, insbesondere 7,9,11,13,15,17,19, 21 und/oder 23.

Weiterhin kann als Kohlenwasserstoff ein Kohlenwasserstoff Gemisch eingesetzt werden, das $^{14}$C- Isotope enthält und wobei das Kohlenwasserstoff Gemisch mindestens 2 voneinander verschiedene Kohlenwasserstoffe enthält, deren C-Zahl sich um mehr als 1 unterscheidet.

Ein weiterer wesentlicher Bestandteil der Mikroemulsionen ist Wasser. Das Wasser sollte vorzugsweise demineralisiert sein. Die Mikroemulsionen enthalten vorzugsweise bis zu 81 Gew.-% Wasser. Bevorzugte Bereiche sind Mengen von 30 bis 80 Gew.-% und insbesondere von 45 bis 65 Gew.-% Wasser.

Neben den oben beschriebene Inhaltsstoffen können die Mikroemulsionen als zusätzlichen Bestandteil noch Fettalkohole der allgemeinen Formel R$^2$-OH enthalten, wobei R$^2$ für einen gesättigten oder ungesättigten, verzweigten oder unverzweigten Alkyl- oder Alkenylrest mit 6 bis 22 C-Atomen steht, enthalten kann. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol. Besonders bevorzugt ist die Mitverwendung von Cetylalkohol, Stearylalkohol Arachylalkohol und Behenylalkohol sowie deren Mischungen.

Wenn Fettalkohole enthalten sind werden sie in Mengen bis zu 15 Gew.-%, bezogen auf die gesamte Mikroemulsion eingesetzt, wobei der Bereich von 1 bis 10 Gew.-% und vorzugsweise 2 bis 8 Gew.-% besonders bevorzugt sein können. Auch diese Fettalkohole, die wasserunlösliche organische Bestandteile darstellen fallen erfindungsgemäß nicht unter die Definition des Ölkörpers.

Die Mikroemulsion, die im ersten Schritt des erfindungsgemäßen Verfahrens hergestellt wird, kann weiterhin noch bis zu 15 Gew.-% Fettalkoholethersulfate enthalten. Typische Beispiele für weitere anionische Tenside sind Seifen, Alkylbenzolsulfonate, Alkansulfonate, Olefinsulfonate, α-Methylestersulfonate, Sulfofettsäuren, Alkylsulfate, Mono- und Dialkylsulfosuccinate, Mono- und Dialkylsulfosuccinamate, Sulfotriglyceride, Monoglyceridsulfate, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate und Proteinfettsäurekondensate (insbesondere pflanzliche Produkte auf Weizenbasis).

Im Sinne der vorliegenden Erfindung sind Fettalkoholethersulfate der allgemeinen Formel R$^3$O-(CH$_2$CH$_2$O)$_m$SO$_3$X, in der R$^3$ für einen linearen oder verzweigten Alkyl- und/oder Alkenylrest mit 6 bis 22 Kohlenstoffatomen, n für Zahlen von 1 bis 10 und X für ein Alkali- und/oder Erdalkalimetall, Ammonium, Alkylammonium, Alkanolammonium oder Glucammonium steht, bevorzugt. Alkylethersulfate ("Ethersulfate") stellen bekannte anionische Tenside dar, die großtechnisch durch SO$_3$- oder Chlorsulfonsäure (CSA)-Sulfatierung von Fettalkohol- oder Oxoalkoholpolyglycolethern und nachfolgende Neutralisation hergestellt werden. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 2 bis 3 Mol Ethylenoxid an technische $C_{12/14}$- bzw. $C_{12/18}$- Kokosfettalkoholfraktionen in Form ihrer Natrium- und/oder Magnesiumsalze.

Die im erfindungsgemäßen Verfahren eingesetzten Mikroemulsionen können noch weitere nichtionische, amphotere und/oder kationische Tenside, vorzugsweise in Mengen von insgesamt 1 bis 25 Gew.-%, bezogen auf das Gesamtgewicht der Emulsion, enthalten. Typische Beispiele für weitere nichtionische Tenside (neben dem Alkyl(oligo)glycosiden) sind z.B. Fettsäure-N-alkylglucamide, Polyolfettsäureester, Zuckerester, Sorbitanester, Polysorbate, Alkoholethoxylate und Aminoxide. Alkoholethoxylate werden herstellungsbedingt als Fettalkohol- oder Oxoalkoholethoxylate bezeichnet und folgen vorzugsweise der Formel $R^4O(CH_2CH_2O)_nH$ $R^4$ für einen linearen oder verzweigten Alkyl- und/oder Alkenylrest mit 6 bis 22 Kohlenstoff-atomen und n für Zahlen von 1 bis 50 steht. Typische Beispiele sind die Addukte von durchschnittlich 1 bis 50, vorzugsweise 5 bis 40 und insbesondere 10 bis 25 Mol an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind Addukte von 10 bis 40 Mol Ethylenoxid an technische Fettalkohole mit 12 bis 18 Kohlenstoffatomen, wie beispielsweise Kokos-, Palm-, Palmkern- oder Talgfettalkohol.

Beispiele für geeignete amphotere bzw. zwitterionische Tenside sind Alkylbetaine, Alkylamidobetaine, Aminopropionate, Aminoglycinate, Imidazoliniumbetaine und Sulfobetaine. Beispiele für geeignete Alkylbetaine stellen die Carboxyalkylierungsprodukte von sekundären und insbesondere tertiären Aminen dar. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, $C_{12/14}$-Kokosalkyldimethylamin, Myristyldimethylamin, Cetyldimethylamin, Stearyldimethyl-amin, Stearylethylmethylamin, Oleyldimethylamin, $C_{16/18}$Talgalkyldimethylamin sowie deren technische Gemische. Weiterhin kommen auch Carboxyalkylierungsprodukte von Amidoaminen in Betracht. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von $C_{8/18}$-Kokosfettsäure-N,N-dime-thylaminopropylamid mit Natriumchloracetat. Weiterhin kommen auch Imidazoliniumbetaine in Betracht. Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum $C_{12/14}$-Kokosfettsäure, die anschließend mit Natriumchloracetat betainisiert werden. Typische Beispiele für kationische Tenside sind quartäre Ammoniumverbindungen und Esterquats, insbesondere quaternierte Fettsäuretrialkanolaminestersalze.

Besonders bevorzugte Mikroemulsionen sind wie folgt zusammengesetzt:

| | |
|---|---|
| Alkyl(oligo)glycoside | 10 bis 20 Gew.-% |
| Monoester des Glycerins von C12-C22-Fettsäuren | 4 bis 10 Gew.-% |
| Ölkörper | 5 bis 30 Gew.-% |
| Fettalkoholethersulfate | 0 bis 15 Gew.-% |
| Fettalkohole | 0 bis 15 Gew.-% |

Der Rest auf 100 Gew.-% ist dann jeweils Wasser, ggf. ergänzt um weitere, optionale Inhaltsstoffe.

Kationische Polymere

Als Komponente b) enthalten die kosmetischen Mittel der vorliegenden Patentanmeldung kationische Polymere. Diese sind bevorzugt gewählt aus der Gruppe der Homo- oder Copolymere von Ester- oder Amidderivaten der Acryl- oder Methacrylsäure (z.B. INCI: Polyquaternium-7), Homopolymeren aus Methacryloylethyltrimethylammonium Chlorid (INCI: Polyquaternium-37), quaternären Copolymeren aus Hydroxyethylcellulose und Diallyl-dimethyl-ammoniumchlorid (INCI: Polyquaternium-4), polymeren quaternisierten Ammoniumsalzen von Hydroxyethylcellulose, welche mit einem trimethylammoniumsubstituierten Epoxid modifiziert sind (INCI: Polyquaternium-10), depolymerisierten Guar Gum Derivaten, welche quaternisiert sind (INCI: Guar Hydroxypropyl Trimonium Chlorid) oder quaternisierte Guarderivate und quaternären Copolymeren aus Hydroxyethylcellulose und Diallyl-demethylammoniumchlorid. In einer bevorzugten Ausführungsform wird das kationische Polymer (c) ausgewählt aus der Gruppe, die gebildet wird von Polyquaternium-7, Polyquaternium-10 und kationischen Guar-Derivaten. Die erfindungsgemäßen Zubereitungen enthalten vorzugsweise 0,05 bis 2 Gew.% dieser kationischen Polymere.

Bevorzugt ist eine kosmetische Zubereitung enthaltend

(a) Alkylethersulfate und Cocamidopropylbetain,
(b) eine Mikroemulsion enthaltend

(b1) 10-20 Gew.-% Alkyloligoglycoside,
(b2) 4-10 Gew.-% Monoester von Glycerin mit einer gesättigten oder ungsättigten Fettsäure der Kettenlänge C12-22 und
(b3) 5-30 Gew.-% Ölkörper und
(b4) Wasser, und

(c) ein kationisches Polymer ausgewählt aus der Gruppe, die gebildet wird von Polyquaternium-7, Polyquaternium-10 und kationischen Guar-Derivaten.

Besonders bevorzugt ist eine kosmetische Zubereitung enthaltend

(a) 5 - 20 Gew.% bezogen auf die Gesamtzusammensetzung Alkylethersulfate und Cocamidopropylbetain,
(b) 0,5 - 10 Gew.% bezogen auf die Gesamtzusammensetzung einer Mikroemulsion enthaltend (Mengenangaben für die Komponenten (b1) bis (b4) bezogen auf die Mikroemulsion)

(b1) 10 - 20 Gew.% Alkyloligoglycoside,
(b2) 4 - 10 Gew.% Monoester von Glycerin mit einer gesättigten oder ungesättigten Fettsäure der Kettenlänge C 12-22,
(b3) 5 - 30 Gew.% eines Ölkörpers und
(b4) Rest auf 100 Gew.% Wasser, und

(c) 0,05 - 1 Gew.% bezogen auf die Gesamtzusammensetzung eines kationischen Polymers ausgewählt aus der Gruppe, die gebildet wird von Polyquaterinium-7, Polyquaternium-10 und kationischen Guar-Derivaten.

Ein weiterer Gegenstand der vorliegenden Erfindung ist ein Verfahren zur Herstellung einer kosmetischen Zubereitung gemäß Anspruch 4.

**Beispiele**

Es wurde zunächst eine Mikroemulsion der folgenden Zusammensetzung durch Vermischen der Inhaltsstoffe hergestellt (Tab. 1 und 2):

Tabelle 1: Zusammensetzung einer Mikromemulsion gemäß Komponente b) der Erfindung:

| Substanz | INCI | Gew.-% Aktivsubstanz |
|---|---|---|
| Plantacare® 2000 UP | Decyl Glucoside | 17,5 |
| Monomuls® 90 O 18 | Glyceryl Oleate | 8 |
| Cetiol® OE | Dicaprylyl Ether | 20 |
| Aqua dem. | | add 100 |

Tabelle 2: Zusammensetzung einer exemplarischen, nicht erfindungsgemäßen Mikromemulsion :

| Substanz | INCI | Gew.-% Aktivsubstanz |
|---|---|---|
| Plantacare® 2000 UP | Decyl Glucoside | 18 |
| Texapon® NSO | Sodium Laureth Sulfate | 12 |
| Cetiol® CC | Dicaprylyl Carbonate | 20 |
| Aqua dem. | | add 100 |

In den folgenden Tabellen 3 und 4 sind verschiedene Shampoo-Formulierungen aufgeführt, die die erfindungsgemäßen Zusammensetzungen aufweisen. Die Beispiele 1-3, 9 und 11 dienen dem Vergleich.

Tabelle 3: Shampoo-Formulierungen und Leistung bei der Nasskämmbarkeit (Mengenangaben in Gew.% Aktivsubstanz)

| Markenprodukt | INCI | 1 | 2 | 3 | 4 | 5 | 6 | 7 | 8 |
|---|---|---|---|---|---|---|---|---|---|
| Texapon® N70 | Sodium Laureth Sulfate | 9 | 9 | 9 | 9 | 9 | 9 | 9 | 9 |
| Dehyton® PK 45 | Cocamidopropyl Betaine | 3 | 3 | 3 | 3 | 3 | 3 | 3 | 3 |
| Emulsion gem. Tab.1 | | 0,00 | 1,78 | 0,00 | 0,36 | 0,89 | 1,78 | 3,57 | 7,14 |
| Dekaquat® 400 KC | Polyquaternium-10 | 0 | 0 | 0,2 | 0,2 | 0,2 | 0,2 | 0,2 | 0,2 |
| Arlypon® TT | PEG/PPG-120/10 Trimethylolpropane | 0,65 | 0,65 | 0,65 | 0,65 | 0,65 | 0,65 | 0,65 | 0,65 |
| Euxyl® K 400 | Methyldibromo Glutaronitrile and Phenoxyethanol | 0,1 | 0,1 | 0,1 | 0,1 | 0,1 | 0,1 | 0,1 | 0,1 |
| Aqua dem. | | add 100 | | | | | | | |
| Restkämmarbeit in % | | 100 | 100 | 58 | 46 | 37 | 29 | 33 | 43 |

Tabelle 4: Shampoo-Formulierungen und Leistung bei der Nasskämmbarkeit (Mengenangaben in Gew.% Aktivsubstanz)

| Markenprodukt | INCI | 9 | 10 | 11 | 12 |
|---|---|---|---|---|---|
| Texapon® N70 | Sodium Laureth Sulfate | 9 | 9 | 9 | 9 |
| Dehyton® PK 45 | Cocamidopropyl Betaine | 3 | 3 | 3 | 3 |
| Emulsion gem. Tab.1 | | 0,00 | 3,57 | 0,00 | 3,57 |
| Jaguar® C 13 S | Guar Hydroxypropyltrimonium Chloride | 0,2 | 0,2 | - | - |
| Rheocare® CC7 | Polyquaternium-7 | - | - | 0,2 | 0,2 |
| Arlypon® TT | PEG/PPG-120/10 Trimethylolpropane | 0,65 | 0,65 | 0,65 | 0,65 |
| Euxyl® K 400 | Methyldibromo Glutaronitrile and Phenoxyethanol | 0,1 | 0,1 | 0,1 | 0,1 |
| NaCl | | 1 | 1 | 1 | 1 |
| Aqua dem. | | add 100 | | | |
| Restkämmarbeit in % | | 83 | 66 | 100 | 81 |

Die Untersuchungen hinsichtlich der Konditionierleistung der Shampoos wurden jeweils an 10 Haarsträhnen in einem automatisierten System zur Bestimmung der Nasskämmarbeit durchgeführt.

Die Vorbehandlung der Haarsträhnen (12cm/lg) der Firma IHIP wurden in einem automatisierten Haarbehandlungssystem durchgeführt:

- 30 min Reinigung mit 6% Natriumlaurylethersulfat, pH 6.5, danach intensives Spülen der Haare
- 20 min Bleiche mit einer Lösung von 5 % Wasserstoffperoxid, pH 9.4 (mit Ammoniumhydroxidlösung eingestellt), danach intensives Spülen der Haare
- 30 min Trocknung in einem Luftstrom bei 68°C

Direkt vor der Nullmessung wurden die Haare für 30 Minuten in Wasser gequollen und anschließend mit einer automatischen Nassauskämmapparatur für 1 Minute ausgespült. Im automatisierten System zur Bestimmung der Nass- und Trockenkämmarbeit wurden die Kämmkräfte während 20 Kämmungen bestimmt und die Kämmarbeit durch Integration der gemessenen Kraft-Weg-Kurven errechnet. Nach der Nullmessung wurden die Haare sofort mit der Formulierung behandelt (0,25g/g Haar). Nach 5 Minuten Einwirkzeit wurde mit der automatischen Nassauskämmapparatur unter Standardbedingungen (38°C, 11/Minute) gespült.

Die Behandlung und das folgende Ausspülen wurde ein zweites Mal wiederholt. Dann erfolgte die Vergleichsmessung (zur Nullmessung). Die Messungen wurden mit der feinen Kammseite der Naturkautschukkämme durchgeführt. Berechnet wurde die Restkämmarbeit pro Strähne wie folgt:

$$\text{Restkämmarbeit} = \text{Kämmarbeit nach Produktbehandlung} / \text{Kämmarbeit vor Produktbehandlung}$$

Anschließend wurde über die Quotienten aller 10 Strähnen der Mittelwert und die Standardabweichung bestimmt.

Die Beispiele belegen eindrucksvoll die synergistische Zusammenwirkung von Polymer und Mikroemulsion, die sich in hervorragend niedrigen Werten für die Restkämmarbeit zeigt.

**Patentansprüche**

1. Kosmetische Zubereitung enthaltend

   (a) mindestens ein Tensid ausgewählt aus anionischen, zwitterionischen oder amphoteren Tensiden,
   (b) eine Mikroemulsion zusammengesetzt aus

   | | |
   |---|---|
   | Alkyl(oligo)glycoside | 10 bis 20 Gew.-% |
   | Monoester des Glycerins von C12-C22-Fettsäuren | 4 bis 10 Gew.-% |
   | Ölkörper | 5 bis 30 Gew.-% |
   | Fettalkoholethersulfate | 0 bis 15 Gew.-% |
   | Fettalkohole | 0 bis 15 Gew.-% |

   auf 100 Gew.-% ist dann jeweils Wasser, ggf. ergänzt um weitere, optionale Inhaltsstoffe, und
   (c) mindestens ein kationisches Polymer.

2. Zubereitung gemäß Anspruch 1, **dadurch gekennzeichnet, dass** das kationische Polymer (c) ausgewählt ist aus der Gruppe, die gebildet wird von Polyquaternium-7, Polyquaternium-10 und kationischen Guar-Derivaten.

3. Zubereitung gemäß einem der Ansprüche 1 bis 2, **dadurch gekennzeichnet, dass** als Komponente (a) eine Mischung von Alkylethersulfat und Cocamidopropylbetain eingesetzt wird.

4. Verfahren zur Herstellung einer kosmetischen Zubereitung, indem

   (a) mindestens ein Tensid ausgewählt aus anionischen, zwitterionischen oder amphoteren Tensiden,
   (b) eine Mikroemulsion zusammengesetzt aus

| Alkyl(oligo)glycoside | 10 bis 20 Gew.-% |
| Monoester des Glycerins von C12-C22-Fettsäuren | 4 bis 10 Gew.-% |
| Ölkörper | 5 bis 30 Gew.-% |
| Fettalkoholethersulfate | 0 bis 15 Gew.-% |
| Fettalkohole | 0 bis 15 Gew.-% |

auf 100 Gew.-% ist dann jeweils Wasser, ggf. ergänzt um weitere, optionale Inhaltsstoffe, und

(c) mindestens ein kationisches Polymer mit weiteren kosmetischen Grundstoffen verrührt.

**Claims**

1. A cosmetic preparation comprising

    (a) at least one surfactant selected from anionic, zwitterionic or amphoteric surfactants,
    (b) a microemulsion composed of

| alkyl (oligo)glycosides | 10 to 20% by weight |
| C12-C22 fatty acid monoesters of glycerol | 4 to 10% by weight |
| oil bodies | 5 to 30% by weight |
| fatty alcohol ether sulfates | 0 to 15% by weight |
| fatty alcohols | 0 to 15% by weight |

then made up to 100% by weight with water in each case, optionally supplemented by further optional ingredients, and
    (c) at least one cationic polymer.

2. The preparation according to claim 1, wherein the cationic polymer (c) is selected from the group formed from polyquaternium-7, polyquaternium-10 and cationic guar derivatives.

3. The preparation according to either of claims 1 and 2, wherein a mixture of alkyl ether sulfate and cocamidopropyl betaine is used as component (a).

4. A method for preparing a cosmetic preparation by stirring

    (a) at least one surfactant selected from anionic, zwitterionic or amphoteric surfactants,
    (b) a microemulsion composed of

| alkyl (oligo)glycosides | 10 to 20% by weight |
| C12-C22 fatty acid monoesters of glycerol | 4 to 10% by weight |
| oil bodies | 5 to 30% by weight |
| fatty alcohol ether sulfates | 0 to 15% by weight |
| fatty alcohols | 0 to 15% by weight |

then made up to 100% by weight with water in each case, optionally supplemented by further optional ingredients, and
    (c) at least one cationic polymer, with further cosmetic base materials.

**Revendications**

1. Préparation cosmétique contenant :

    (a) au moins un tensioactif choisi parmi les tensioactifs anioniques, zwitterioniques ou amphotères,

(b) une microémulsion composée de

| | |
|---|---|
| (oligo)glycosides d'alkyle | 10 à 20 % en poids |
| monoesters de glycérine d'acides gras en C12-C22 | 4 à 10 % en poids |
| corps gras | 5 à 30 % en poids |
| éther-sulfates d'alcools gras | 0 à 15 % en poids |
| alcools gras | 0 à 15 % en poids |

puis à chaque fois de l'eau jusqu'à 100 % en poids, éventuellement supplémentée avec d'autres composants optionnels, et
(c) au moins un polymère cationique.

2. Préparation selon la revendication 1, **caractérisée en ce que** le polymère cationique (c) est choisi dans le groupe formé par le polyquaternium 7, le polyquaternium 10 et les dérivés de guar cationiques.

3. Préparation selon l'une quelconque des revendications 1 à 2, **caractérisée en ce qu'**un mélange d'éther-sulfate d'alkyle et de cocamidopropylbétaïne est utilisé en tant que composant (a).

4. Procédé de fabrication d'une préparation cosmétique, selon lequel

(a) au moins un tensioactif choisi parmi les tensioactifs anioniques, zwitterioniques ou amphotères,
(b) une microémulsion composée de

| | |
|---|---|
| (oligo)glycosides d'alkyle | 10 à 20 % en poids |
| monoesters de glycérine d'acides gras en C12-C22 | 4 à 10 % en poids |
| corps gras | 5 à 30 % en poids |
| éther-sulfates d'alcools gras | 0 à 15 % en poids |
| alcools gras | 0 à 15 % en poids |

puis à chaque fois de l'eau jusqu'à 100 % en poids, éventuellement supplémentée avec d'autres composants optionnels, et
(c) au moins un polymère cationique

sont mélangés avec d'autres matériaux de base cosmétiques.

EP 2 164 449 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004030885